# EUROPÄISCHE PATENTSCHRIFT

(11) **EP 0 305 889 B1**
(45) Veröffentlichungstag und Bekanntmachung des Hinweises auf die Patenterteilung: **22.02.1995**
(21) Anmeldenummer: 88113830.9
(22) Anmeldetag: 25.08.1988
(51) Int. Cl.: C07D 471/04, C07B 41/04

(54) **Verfahren zur Herstellung von 4-Alkoxyalkyl-beta-carbolinen**
Process for the preparation of 4-alkoxyalkyl-beta-carbolines
Procédé pour la préparation d'alkoxyalkyl bêta-carbolines

(30) Priorität: 31.08.1987 DE 3729370
(43) Veröffentlichungstag der Anmeldung: 08.03.1989
(73) Patentinhaber: SCHERING AKTIENGESELLSCHAFT, 13342 Berlin (DE)
(72) Erfinder: Huth, Andreas, Dr., D-1000 Berlin 38 (DE); Rahtz, Diether, Dr., D-1000 Berlin 38 (DE); Rohde, Ralph, Dr., D-1000 Berlin 45 (DE); Schmiechen, Ralph, Dr., D-1000 Berlin 42 (DE); Seidelmann, Dieter, Dr., D-1000 Berlin 41 (DE)

(56) Entgegenhaltungen:
- EP-A- 234 173
- US-A- 4 062 842
- HETEROCYCLES (1983) 20, PP.1295-1313
- ANGEWANDTE CHEMIE, 69. Jahrgang, Nr. 13/14, 1957, Weinheim; H. MEERWEIN "Alkylierung mit Halogenalkylen und Silberfluoroborat", seite 481.

## Beschreibung

Die Erfindung betrifft ein Verfahren zur Herstellung von 4-Alkoxyalkyl-β-carbolinderivaten.

β-Carboline, die in 4-Stellung mit einer Alkoxyalkyl-Gruppe substituiert sind, zeigen eine sehr gute Bindungsaffinität an die Benzodiazepinrezeptoren wie in früheren Anmeldungen beschrieben ist, beispielsweise in den EP-A-54 507,-161575,- 234173,- 130140.

Nach dem vorbekannten Verfahren wird die Alkoxyalkylgruppe schon auf einem ganz frühen Reaktionsschritt eingeführt, so daβ für die Herstellung von alkoxyalkylierten Verbindungen die gesamte β-Carbolin-Synthese durchlaufen werden muß. Diese Synthese ist auch dadurch erschwert, daβ die eingesetzten Aldehyde in schwierig handhabbaren Synthesen selbst hergestellt werden müssen. Nur der Methoxyacetaldehyd ist käuflich.

Es stellte sich daher die Aufgabe, ein breit anwendbares Verfahren zur Herstellung von 4-Alkoxyalkyl-β-carbolinen zu entwickeln, das in einfacher Weise mit guten Ausbeuten und in Gegenwart nicht toxischer Verbindungen die Einführung des Alkoxyalkyl-Restes an diversen β-Carbolinen ermöglicht.

Die naheliegende Veretherung der entsprechenden Halogenalkyl-Verbindung mit Alkoholaten führte hauptsächlich zu unerwünschten Kondensationsprodukten und gelang nur mit der schwerer zugänglichen Jodalkylverbindung und dann auch nur mit Ethanol in schlechter Ausbeute, wie Het. (1983) 20 (7), 1295-1313 entnommen werden kann.

Fügt man der alkoholischen Lösung des β-Carbolins jedoch Silbertetrafluoroborat als Katalysator zu, so gelingt die Veretherung in sehr guter Ausbeute und Reinheit des Produktes auf einfachem Wege mit hoher Variationsbreite und ohne aufwendige Trennoperationen oder Hinzufügung toxischer Reaktionszusätze.

Über die O-Alkylierung in Gegenwart von Silbersalzen wird beispielsweise in Angew. Chemie (1957) 69, 481, US-A-4062842 und Merck Schuchardt 015 Info 84-4 berichtet. Daraus ist zu entnehmen, daβ bei Verwendung von Silbertetrafluoroborat häufig Umlagerungen auftreten.

Es war daher überraschend, daß unter den beanspruchten Reaktionsbedingungen die Veretherung mit aliphatischen Alkoholen in guten Ausbeuten an 4-Halogenalkyl-β-carbolinderivaten in einem Reaktionsschritt gelingt, wobei auch sterisch gehinderte Alkohole wie tert. Butanol und sich leicht umlagernde Alkohole wie Cyclobutanol und Cyclopropylmethanol umgesetzt werden können.

Die Erfindung betrifft ein Verfahren zur Herstellung von 4-Alkoxyalkyl-β-carbolinderivaten der allgemeinen Formel I 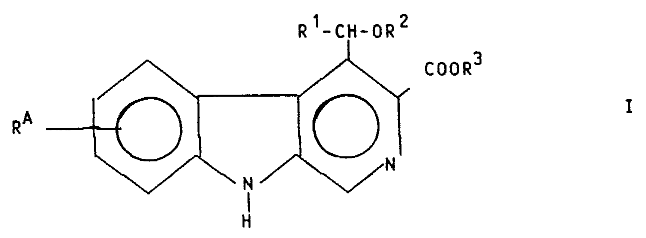
worin
- R¹: Wasserstoff oder Methyl,
- R²: einen geradkettigen oder verzweigten C₁₋₆-Alkyl-, Alkenyl- bzw. Alkinylrest mit bis zu 6 Kohlenstoffatomen, der jeweils ein- bis dreifach mit Halogen oder C₁₋₂-Alkoxy substituiert sein kann oder eine C₃₋₇-Cycloalkyl- oder Cycloalkylalkylgruppe,
- R³: C₁₋₄-Alkyl und
- R^{A}: Wasserstoff, Halogen, COOR⁴ oder OR⁵ bedeutet und ein- oder zweifach stehen kann, wobei
- R⁴: C₁₋₄-Alkyl und
- R⁵: Wasserstoff, C₁₋₄-Alkyl, C₃₋₇-Cycloalkyl oder Phenyl ist, das mit Halogen, C₁₋₄-Alkyl oder C₁₋₄-Alkoxy substituiert sein kann,
dadurch gekennzeichnet, daß man eine Verbindung der allgemeinen Formel II 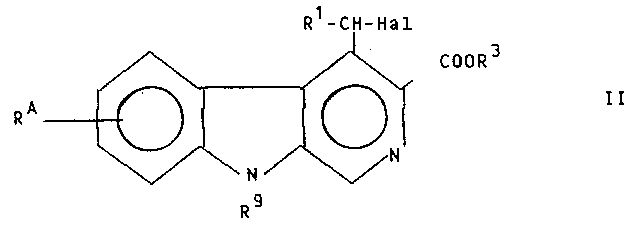
worin
- R¹ und R³: die obige Bedeutung haben und
- R^{A}: Wasserstoff, Halogen, COOR⁴ oder OR^{5'} ist, wobei
- R⁴: C₁₋₄-Alkyl und
- R^{5'}: R⁵ oder C₁₋₄-Alkanoyl ist, und
- Hal: Halogen und
- R⁹: eine Schutzgruppe darstellt,
in Gegenwart von Ag B F₄ mit einem Alkohol der Formel R²OH mit R² in der oben genannten Bedeutung umsetzt und gegebenenfalls die Schutzgruppe abspaltet.

Als aliphatischer Kohlenwasserstoffrest R² kommen geradkettige und verzweigte gegebenenfalls substituierte Alkylgruppen mit 1-6 Kohlenstoffatomen und geradkettige und verzweigte gegebenenfalls substituierte Alkenyl- bzw. Alkinylgruppen mit bis zu 6 Kohlenstoffatomen in Betracht, ferner Cycloalkyl- und Cycloalkylalkylgruppen mit 3-7 Kohlenstoffatomen.

Geeignete gesättigte Alkylgruppen sind beispielsweise Methyl, Ethyl, Propyl, Isopropyl, Butyl, Isobutyl, sek. Butyl, tert. Butyl, Pentyl, 2-Methylbutyl, 2,2-Dimethylpropyl und Hexyl.

Als ungesättigte Alkylgruppen seien vorzugsweise die nachfolgenden Alkenyle und Alkinyle mit 3-5 Kohlenstoffatomen genannt: 2-Propenyl, 3-Methyl-2-propenyl, 2-Propinyl, Methallyl.

Als Substituenten kommen Halogene insbesondere Fluor und Chlor sowie C₁₋₂ Alkoxy-Gruppen in Betracht, wobei der Substituent 1-3fach auftreten kann.

Bedeutet der Kohlenwasserstoffrest R² eine Cycloalkylgruppe, so seien beispielsweise Cyclopropyl, Cyclobutyl, Cyclopentyl, Cyclohexyl und Cycloheptyl als bevorzugt genannt.

Als Cycloalkyl-alkylgruppe R² kommt beispielsweise die Cyclopropylmethyl-, Cyclopropylethyl- und die Cyclopentylmethylgruppe in Betracht.

Als niedere Alkylgruppen R³, R⁴ und R⁵ sind beispielsweise die oben genannten gesättigten Alkylreste mit 1-4 Kohlenstoffatomen geeignet.

Der Substituent R^{A} kann 1-2fach in 5, 6- oder 7-Stellung am β-Carbolin stehen, wobei die 5-oder 6-Stellung bevorzugt ist.

Als Cycloalkylreste R⁵ kommen die für R² genannten Cycloalkylreste in Frage.

Bedeutet R⁵ einen substituierten Phenylrest so seien als bevorzugt Halogen, niederes Alkyl und niederes Alkoxy genannt.

Der Substituent R⁹ stellt eine üblicherweise verwendete Schutzgruppe dar, wie beispielsweise eine Acyl-, Arylsulfonyl- oder Silyl-Gruppe wie die Trialkylsilyl-Gruppe.

Als niederer Acylrest R⁵ sind Säuren mit bis zu 4-Kohlenstoffatomen geeignet. Insbesondere kommt der Acetylrest in Betracht der bei der Veretherung verseifend abgespalten wird.

Als Halogen sind Fluor, Chlor, Brom und Jod und in der Halogenalkyl-Verbindung der allgemeinen Formel II insbesondere Brom und Chlor geeignet.

Die Veretherung der Verbindungen der allgemeinen Formel II erfolgt bei Temperaturen von Raumtemperatur bis zur Siedetemperatur des Lösungsmittels und ist im allgemeinen nach 1 bis 3 Stunden beendet. Als Lösungsmittel sind insbesondere die entsprechenden Alkohole und auch Gemische der Alkohole beispielsweise mit Kohlenwasserstoffen, Ethern und chlorierten Kohlenwasserstoffen geeignet.

Zweckmäßigerweise wird unter Inertgasatmosphäre, beispielsweise unter Stickstoff oder Argon, gearbeitet.

Silbertetrafluoroborat wird als Katalysator in molarer Menge oder in einem geringen Überschuß der Reaktionslösung hinzugefügt.

Die Schutzgruppe wird unter den Reaktionsbedingungen abgespalten oder kann anschließend nach den üblichen Methoden, beispielsweise durch Behandeln mit Basen wie Natrium- oder Kaliumalkoholat oder Säuren wie verdünnter Mineralsaure oder Trifluoressigsäure in inerten Lösungsmitteln wie Alkoholen, Kohlenwasserstoffen u.a. bei Raumtemperatur abgespalten werden.

Die so erhaltenen Verbindungen der allgemeinen Formel I sind wirksame Pharmaka oder können, insbesondere falls R^{A} = OH bedeutet, zu sehr gut wirksamen Pharmaka nach bekannten Methoden umgesetzt werden (beispielsweise EP-A-130 140, EP-A-237467, EP-A 239667)
Die Herstellung der Ausgangsverbindungen erfolgt in einfacher Weise beispielsweise nach der nachfolgend beschriebenen Methode:
9-Acetyl-4-brommethyl-β-carbolin-3-carbonsäureethylester (G. Neef et al. Het. 20 (7), 1295 (1983)).

10 g (33,8 mMol) 9-Acetyl-4-methyl-β-carbolin-3-carbonsäureethylester werden in 500 ml Tetrachlorkohlenstoff mit 12 g (67,4 mMol) N-Bromsuccinimid und 1 Spatelspitze Azobisisobutyronitril versetzt und 1,5 h unter gleichzeitiger Belichtung zum Rückfluß erhitzt. Nach Abkühlen wird vom Succinimid abgesaugt, eingeengt und der Ruckstand mit heißem Wasser ausgerührt und abgesaugt. Man erhält
10,64 g (84 %) 9-Acetyl-4-brommethyl-β-carbolin-3-carbonsäureethylester vom Schmelzpunkt 188-189°C.

In analoger Weise werden aus den in den EP-A 54 507, 128415, 161575 und 234173 beschriebenen Verbindungen die 4-Brommethyl-Verbindungen hergestellt.

Beispielsweise seien genannt:
9-Acetyl-4-brommethyl-5-isopropoxy-β-carbolin-3-carbonsäureethylester in 74,3 % Ausbeute
9-Acetyl-5-acetoxy-4-brommethyl-β-carbolin-3-carbonsäureethylester in 60 % Ausbeute
9-Acetyl-4-brommethyl-6-phenoxy-β-carbolin-3-carbonsäureethylester in 90 % Ausbeute.

Die nachfolgenden Beispiele sollen das erfindungsgemäße Verfahren erläutern.

### Allgemeine Arbeitsvorschrift:

### 4-Methoxymethyl-β-carbolin-3-carbonsäureethylester

372 mg (1 mMol) 4-Brommethyl-9-acetyl-β-carbolin-3-carbonsäureethylester werden unter Argon in 20 ml Methanol auf 50°C erwärmt und unter 390 mg (2mMol) Silbertetrafluoroborat versetzt. Danach wird eine Stunde am Rückfluß gekocht. Nach Stehen über Nacht wird über Kieselgur abgesaugt und mit Methanol nachgewaschen. Nach Einengen des Filtrates wird in Methylenchlorid und verdünntem wässrigen Ammoniak verteilt, die wässrige Phase zweimal unter Methylenchlorid ausgeschüttelt und die gesammelte organische Phase mit Wasser gewaschen. Nach Trocknen über Sikkon®, Filtrieren und Einengen wird über Kieselgel chromatographiert. Zunächst mit Toluol: Eisessig:Wasser = 10:10:1

Nach Zusammenfassen und Einengen der entsprechenden Fraktionen wird in Essigester/verdünntem Ammoniak verteilt.

Man erhalt 120 mg (42,3 %) 4-Methoxymethyl-β-carbolin-3-carbonsäureethylester vom Schmelzpunkt 104-106°C.

In analoger Weise erhält man die in der Tabelle aufgeführten Verbindungen.

| R^{A} | R¹ | R² | R³ | Schmelzpunkt °C |
|---|---|---|---|---|
| H | H | Me | Et | 104-106 |
| H | H | Et | Et | 126-129 |
| H | H | n-Prop | Et | 195-197 |
| H | H | n-But | Et | 103-105 |
| H | H | i-Prop | Et | 170-173 |
| H | H | tert. But | Et | 162-163 |
| H | H | Allyl | Et | 129-131 |
| H | H | CH₂-CH₂-OCH₃ | Et | 88-90 |
| H | H | CH₂-CF₃ | Et | 190-192 |
| H | CH₃ | Et | Et | 171-173 |
| H | H | Cyclobutyl | i-Prop | 204-205 |
| H | H | Cyclopropylmethyl | Et | 160-161 |
| OH | H | Prop | Et | 188-190 |
| Phenoxy | H | Prop | i-Prop | 171-172 |
| O-i-Prop | H | n-Prop | Et | 203-204 |

## Patentansprüche

1. Verfahren zur Herstellung von 4-Alkoxyalkyl-β-carbolinderivaten der allgemeinen Formel I 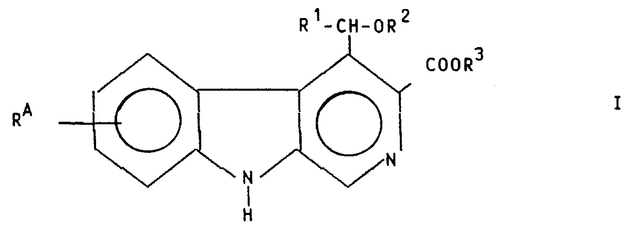 worin
R¹ Wasserstoff oder Methyl,
R² einen geradkettigen oder verzweigten C₁₋₆-Alkyl, Alkenyl- bzw. Alkinylrest mit bis zu 6 Kohlenstoffatomen, der jeweils ein- bis dreifach mit Halogen oder C₁₋₂-Alkoxy substituiert sein kann oder eine C₃₋₇-Cycloalkyl- oder Cycloalkylalkylgruppe,
R³ C₁₋₄-Alkyl und
R^{A} Wasserstoff, Halogen, COOR⁴ oder OR⁵ bedeutet und ein- oder zweifach stehen kann, wobei
R⁴ C₁₋₄-Alkyl und
R⁵ Wasserstoff, C₁₋₄-Alkyl, C₃₋₇-Cycloalkyl oder Phenyl ist, das mit Halogen, C₁₋₄-Alkyl oder C₁₋₄-Alkoxy substituiert sein kann,
dadurch gekennzeichnet, daß man eine Verbindung der allgemeinen Formel II 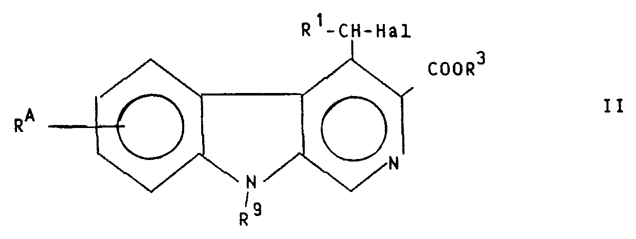 worin
R¹ und R³ die obige Bedeutung haben und
R^{A} Wasserstoff, Halogen, COOR⁴ oder OR^{5'} ist, wobei
R⁴ C₁₋₄-Alkyl und
R^{5'} R⁵ oder C₁₋₄-Alkanoyl ist, und
Hal Halogen und
R⁹ eine Schutzgruppe darstellt,
in Gegenwart von Ag B F₄ mit einem Alkohol der Formel R²OH mit R² in der oben genannten Bedeutung umsetzt und gegebenenfalls die Schutzgruppe abspaltet.

## Claims

1. Process for the preparation of 4-alkoxyalkyl-β-carboline derivatives of the general formula I 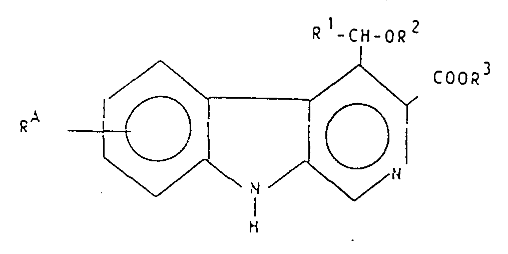 wherein
R¹ is hydrogen or methyl,
R² is a straight-chained or branched C₁₋₆alkyl, alkenyl respectively alkynyl radical having up to 6 carbon atoms, each of which may be mono-, di- or tri-substituted by halogen or by C₁₋₂alkoxy, or is a C₃₋₇cycloalkyl or C₃₋₇cycloalkylalkyl group,
R³ is C₁₋₄alkyl and
R^{A} is hydrogen, halogen, COOR⁴ or OR⁵ and may be present once or twice, wherein
R⁴ is C₁₋₄alkyl and
R⁵ is hydrogen, C₁₋₄alkyl, C₃₋₇cycloalkyl, or phenyl that may be substituted by halogen, C₁₋₄alkyl or by C₁₋₄alkoxy,
characterised in that a compound of the general formula II 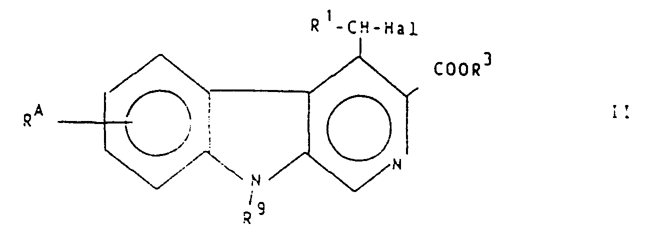 wherein
R¹ and R³ are as defined above and
R^{A} is hydrogen, halogen, COOR⁴ or OR^{5'}, wherein
R⁴ is C₁₋₄alkyl and
R^{5'} is R⁵ or C₁₋₄alkanoyl, and
Hal is halogen and
R⁹ is a protecting group,
is reacted in the presence of AgBF₄ with an alcohol of formula R²OH in which R² is as defined above, and the protecting group is optionally removed.

## Revendications

1. Procédé pour la préparation de dérivés de 4-alcoxyalkyl-β-carboline de formule générale I 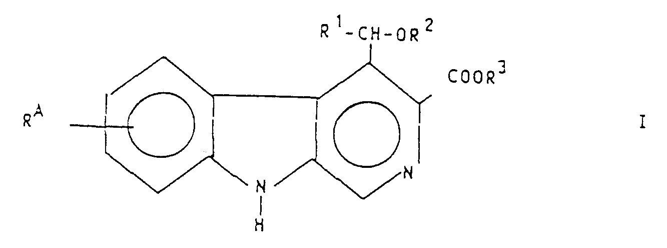 où
R¹ représente l'hydrogène ou le méthyle,
R² représente les radicaux alkyle en C₁₋₆, alcényle, respectivement alcynyle, avec jusqu'à 6 atomes de carbone, linéaires ou ramifiés, qui peuvent être chaque fois substitués 1 à 3 fois par des halogènes ou alcoxy en C₁₋₂ ou un groupe cycloalkylalkyle ou cycloalkyle en C₃₋₇,
R³ représente un alkyle en C₁₋₄, et
R^{A} représente l'hydrogène, les halogène, COOR⁴ ou OR⁵ et peut être présent une ou deux fois,
R⁴ représentant un alkyle en C₁-₄, et
R⁵ représentant l'hydrogène, les alkyle en C₁₋₄, cycloalkyle en C₃₋₇ ou phényle, qui peut être substitué par des halogène, alkyle en C₁₋₄ ou alcoxy en C₁₋₄,
caractérisé en ce qu'on fait réagir un composé de formule générale II 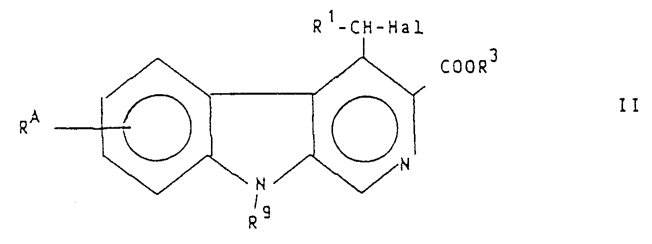 où
R¹ et R² ont la signification donnée ci-dessus et
R^{A} représente l'hydrogène, les halogène, COOR⁴ ou OR^{5'},
R⁴ représentant un alkyle en C₁₋₄ et
R^{5'} représentant R⁵ ou un alcanoyle en C₁-₄, et
Hal représente un halogène et
R⁹ représente un groupe protecteur,
en présence de AgBF₄, avec un alcool de formule R²OH avec R² ayant la signification donnée ci-dessus et éventuellement, on sépare le groupe protecteur.
